# FASCICULE DE BREVET EUROPEEN

(11) **EP 0 673 649 B1**
(45) Date de publication et mention de la délivrance du brevet: **24.04.2002**
(21) Numéro de dépôt: 95400628.4
(22) Date de dépôt: 22.03.1995
(51) Int. Cl.: A61K 31/495, A61K 9/28, A61K 9/50

(54) **Compositions pharmaceutiques permettant la libération prolongée de trimétazidine après administration par voie orale**
Arzneizubereitungen für die verlängerte Trimetazidinfreisetzung nach oraler Verabreichnung
Pharmaceutical compositions for the prolonged release of trimetazidine after oral administration

(30) Priorité: 24.03.1994 FR 9403434
(43) Date de publication de la demande: 27.09.1995
(73) Titulaire: LES LABORATOIRES SERVIER, 92200 Neuilly sur Seine (FR)
(72) Inventeur: Huet de Barochez, Bruno, F-45520 Chevilly (FR); Genty, Patrick, F-45000 Orleans (FR); Cuine, Alain, F-45800 Saint Jean de Braye (FR)

(56) Documents cités:
- GB-A- 2 084 019
- DATABASE WPI Week 8644, Derwent Publications Ltd., London, GB; AN 86-289031 (44) & JP-A-61 212 517 (NIPPON CHEMIFAR) 20 Septembre 1986

## Description

La présente invention a pour objet une composition pharmaceutique permettant la libération prolongée de trimétazidine ou d'un de ses sels d'addition à un acide pharmaceutiquement acceptable.

La trimétazidine, ou 1-(2,3,4-triméthoxybenzyl)pipérazine, composé de formule : 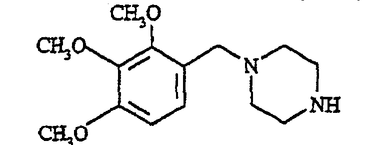 est une molécule qui, en préservant les métabolismes énergétiques de la cellule exposée à l'hypoxie ou à l'ischémie, évite l'effondrement du taux intracellulaire de l'adénosine triphosphate (ATP). Elle assure ainsi le fonctionnement des pompes ioniques et des flux transmembranaires sodium - potassium et maintient l'homéostasie cellulaire.

Parmi les sels pharmaceutiquement acceptables de trimétazidine, on citera plus particulièrement la dichlorhydrate qui est utilisé en thérapeutique, comme vasodilatateur coronarien pour le traitement prophylactique de la crise d'angine de poitrine, lors des atteintes choriorétiniennes ainsi que pour le traitement des vertiges d'origine vasculaire (vertiges de Ménière, acouphènes).

Le dichlorhydrate de trimétazidine était jusqu'à présent administré par voie orale à des doses de 40 à 60 mg par jour, sous forme de comprimés dosés à 20 mg, ou de solution buvable dosée à 20 mg/ml. Ces deux formes sont des formes galéniques à libération immédiate.

Le dichlorhydrate de trimétazidine est rapidement absorbé et éliminé par l'organisme, sa demi-vie plasmatique étant inférieure à 6 heures. Ceci impose de fractionner l'administration du principe actif en 2 ou 3 prises par jour afin d'assurer des taux plasmatiques relativement constants. Mais, du fait d'une absorption rapide, ces formes à libération immédiate conduisent également à des pics plasmatiques importants quelques instants après administration et à des taux sanguins très faibles au moment de la prise suivante.

Une forme à libération prolongée présente donc l'avantage de diminuer ces pics sanguins en assurant des taux sanguins réguliers et constants. Elle permet également une administration journalière unique ce qui est favorable à la compliance du patient à son traitement. Elle permet donc un meilleur suivi du traitement.

Pour ce faire, il est nécessaire d'assurer une libération prolongée dans le temps, de façon parfaitement contrôlée. La vitesse de libération doit être reproductible et corrélée avec les concentrations sanguines observées après administration.

Parmi les mécanismes pouvant être invoqués pour le contrôle de la diffusion d'un principe actif soluble, on peut en retenir un principal qui est la diffusion du principe actif à partir d'un réservoir et à travers un film polymérique lorsque la forme galénique est mise au contact du liquide de dissolution (in vitro), ou du liquide gastro-intestinal (in vivo).

De nombreux polymères ont été décrits comme pouvant permettre une telle diffusion. Les principaux sont l'éthylcellulose et les dérivés métacryliques. Les procédés de fabrication couramment utilisés pour la fabrication de tels systèmes réservoirs font appel à des enrobages: soit enrobage de comprimés nus, la dose de principe actif étant alors répartie dans une seule unité, soit enrobage de minigranules ; dans ce dernier cas, la dose de principe actif à administrer est répartie dans de nombreuses petites unités regroupées ensuite soit dans une gélule, soit dans un comprimé.

Afin de former un film barrière permettant la libération prolongée du principe actif, les polymères utilisés sont des polymères insolubles dans l'eau et les liquides gastro-intestinaux. Ils peuvent être utilisés soit sous forme de solution organique, soit sous forme de dispersion aqueuse (latex ou pseudo-latex). Des procédés utilisant la compression ont également été décrits.

La composition pharmaceutique décrite dans la présente invention est caractérisé par le fait que la libération prolongée est assurée par le système réservoir qui associe au principe actif présenté soit sous forme de comprimés, soit sous forme de minigranules, un film de polymère d'épaisseur parfaitement contrôlée enrobant individuellement chaque comprimé ou minigranule, afin d'obtenir une libération du principe actif conduisant à des taux plasmatiques parfaitement définis.

De nombreuses études ont été réalisées sur le contrôle de la libération de principes actifs très hydrosolubles. Beaucoup se sont heurtées au problème suivant: lorsque le principe actif est d'une taille relativement réduite (PM < 300) et de plus très solubles dans l'eau, le contrôle de la libération du principe actif est relativement complexe, et en particulier, il n'est pas possible d'assurer une libération du principe actif sur plus de 12 heures.

Des travaux antérieurs nous ont montré qu'il en était de même avec le dichlorhydrate de trimétazidine.

Le poids moléculaire de la trimétazidine sous forme de base est de 266,33 et la solubilité du dichlorhydrate dans l'eau supérieure à 1000 mg/ml. De ce fait, il était très difficile d'obtenir des comprimés matriciels contrôlant la libération du principe actif sur plus de 16 heures. Ce problème a été résolu par l'utilisation du système réservoir décrit dans la présente invention.

Il nous est apparu que la combinaison d'un polymère insoluble dans l'eau, tel que l'éthylcellulose ou un polyméthacrylate, avec un plastifiant permettant l'obtention d'un film relativement perméable au principe actif permettait d'obtenir une libération régulière de ce dernier.

L'influence d'un plastifiant sur la perméabilité de films de polyméthacylate (Eudragit RS® ) mesuré avec du chlorhydrate de phénylpropanolamine a été décrite par K. LEHMAN (dans "Aqueous polymeric coatings for pharmaceutical dosage forms", Ed. J.W. Mc Ginity, Marcel Dekker, Inc., NY, 1989, p.205). Les résultats obtenus par K. LEHMAN sont regroupés dans le tableau ci-dessous.

| Plastifiant (ajouté à 20 %) | Perméabilité (mg.cm⁻².h⁻¹) | Solubilité dans l'eau à température ambiante (%) |
|---|---|---|
| Acétyltriéthylcitrate | 3.8 | 0.72 |
| Dibutylphtalate | 5.4 | 0.04 |
| Triéthylcitrate | 6.2 | 6.9 |
| Triacétine | 6.2 | 7.1 |
| Tributylcitrate | 9.0 | <0.002 |
| Acétyltributylcitrate | 11.8 | <0.002 |
| Diéthylphtalate | 14.8 | 0.15 |

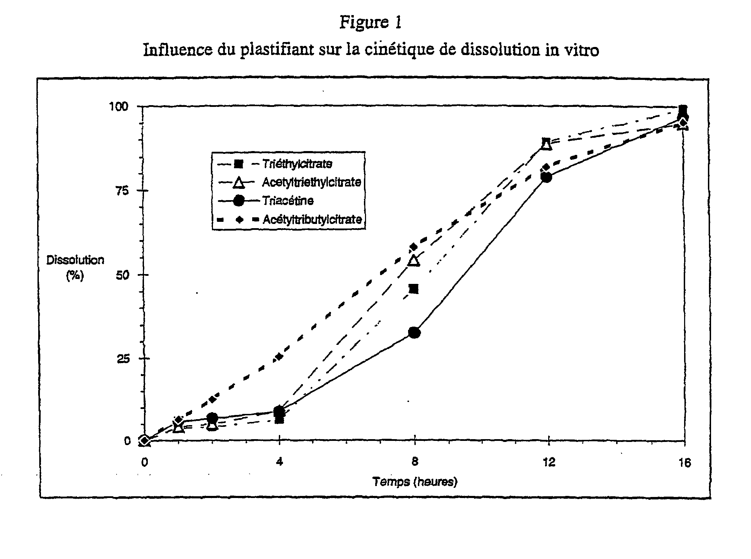

Au vu de la solubilité très importante du dichlorhydrate de trimétazidine et des résultats présentés dans le tableau précédent, il apparaissait judicieux d'utiliser les plastifiants ayant la plus faible perméabilité. Or, de façon surprenante, il a été montré que les meilleurs résultats ont été obtenus avec l'acétyltributylcitrate.
En effet, l'utilisation de plastifiants comme l'acétyltriéthylcitrate, le triéthylcitrate ou la triacétine, provoque un temps de latence très long qui peut atteindre quatre heures au bout desquelles la libération est beaucoup plus rapide qu'avec l'acétyltributylcitrate. Ces résultats sont présentés dans la figure 1 (annexe).
Pour les traitements des désordres coronariens auxquels la trimétazidine s'adresse, il était très important que la libération du principe actif puisse commencer dès l'administration de la forme galénique au malade, de manière à lui assurer un traitement rapide et efficace. Un temps de latence supérieur à une heure est donc un handicap que la présente invention a su surmonter. De plus, une libération progressive du principe actif est indispensable si l'on veut obtenir des taux plasmatiques relativement constants.

Les compositions pharmaceutiques décrites dans la présente invention se présentent plus particulièrement sous forme de comprimés ou de minigranules réservoir.

### Principe de fabrication des formes galéniques

### Principe de fabrication des comprimés réservoir

Les comprimés sont préparés en plusieurs étapes.

Tout d'abord un mélange préalable des constituants permet d'obtenir une régularité du dosage final en dichlorhydrate de trimétazidine. Une phase de granulation permet ensuite de densifier le mélange de poudre initial. Cette agglomération peut se faire à l'aide d'un liant soluble dans un solvant aqueux ou hydroalcoolique. Une phase de lubrification permet ensuite d'autoriser un bon fonctionnement des machines à produire les comprimés.

Après obtention des comprimés, ces derniers sont enrobés au moyen d'une solution ou d'une suspension du polymère choisi comme devant assurer la diffusion du principe actif et contrôler ainsi la cinétique de libération. Un plastifiant est utilisé à ce stade afin de réaliser un bon recouvrement du comprimé par le film polymérique.

### Principe de fabrication des minigranules réservoir

Les minigranules peuvent être préparés suivant deux procédés, soit un procédé utilisant des techniques d'extrusion et de sphéronisation, soit un procédé utilisant des techniques de montage du principe actif sur un noyau neutre.

### a - Fabrication de minigranules par extrusion et sphéronisation

Dans ce procédé, une masse humide renfermant le dichlorhydrate de trimétazidine et des excipients auxiliaires est extrudée à travers une grille perforée. Le produit obtenu sous forme de cylindres allongés est ensuite sphéronisé au moyen d'un sphéroniseur à plateau denté. Les minigranules ainsi obtenus sont par la suite séchés, soit en étuve ventilée, soit en lit d'air fluidisé.

### b - Fabrication de minigranules par montage du principe actif sur un novau neutre

Dans ce procédé, des noyaux neutres sont enrobés par des couches successives de principe actif au moyen de turbine d'enrobage, perforée ou non, ou d'appareils à lit d'air fluidisé. Le principe actif, mis sous forme de solution ou suspension, aqueuse ou organique, est pulvérisé sur les noyaux neutres puis séché par de l'air chaud par exemple.

### c - Enrobage des minigranules

Les minigranules, préparés par l'un ou l'autre des procédés sont ultérieurement enrobés, soit en turbine d'enrobage, perforée ou non, soit en appareil de type lit d'air fluidisé. Les minigranules sont enrobés au moyen d'une solution ou d'une suspension du polymère choisi comme devant assurer la diffusion du principe actif et contrôler ainsi la cinétique de libération. Un plastifiant est utilisé à ce stade afin de réaliser un bon recouvrement des minigranules par le film polymérique.

Les exemples suivants illustrent l'invention mais ne la limitent en aucune façon.

### Comprimés

La préparation des comprimés à libération prolongée est réalisée de la manière suivante :

### Stade A

Mélange du dichlorhydrate de trimétazidine, d'un diluant et de polyvidone agissant comme liant puis mouillage au moyen d'une solution aqueuse ou hydroalcoolique. La masse humide est ensuite granulée, séchée puis calibrée de manière à obtenir un granulé dont les caractéristiques physiques permettent un bon remplissage des matrices d'une machine à comprimer rapide.

### Stade B

Lubrification du granulé obtenu au stade A avec de la silice colloïdale et/ou du stéarate de magnésium.

### Stade C

Compression du mélange lubrifié obtenu au stade B sur une machine à comprimer alternative ou rotative.

### Stade D

Enrobage des comprimés obtenus au stade C au moyen d'une solution ou d'une suspension, aqueuse ou organique, d'un polymère assurant la diffusion du principe actif. Cette solution ou suspension peut également renfermer un plastifiant. L'enrobage est réalisé dans une turbine d'enrobage ou un appareil à lit d'air fluidisé.

### Exemple 1 :

Un comprimé à libération prolongée (LP1) est préparé en utilisant la formule donnée dans le tableau 2, suivant le procédé opératoire décrit dans les stades A à C.

**Tableau 2**

| Formule unitaire du comprimé LP1 | |
|---|---|
| Constituants | Quantités (mg) |
| Dichlorhydrate de trimétazidine | 80 |
| Hydrogénophosphate de calcium | 80 |
| Polyvidone | 8 |
| Silice colloïdale | 0.4 |
| Stéarate de magnésium | 1.6 |

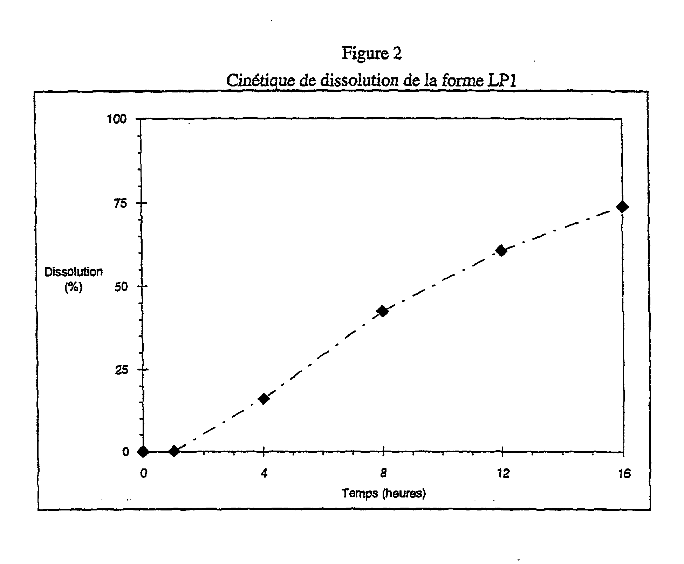

Ce comprimé est ensuite enrobé en turbine perforée au moyen d'une solution alcoolique d'éthylcellulose renfermant de l'acétyltributylcitrate utilisé comme plastifiant. Le profil de dissolution in vitro de cette forme (LP1) est présenté dans la figure 2 (annexe).

Le temps de latence de cette forme à libération prolongée est égal à une heure. Au-delà, la cinétique de dissolution in vitro est linéaire sur plus de 10 heures.

### Exemple 2 :

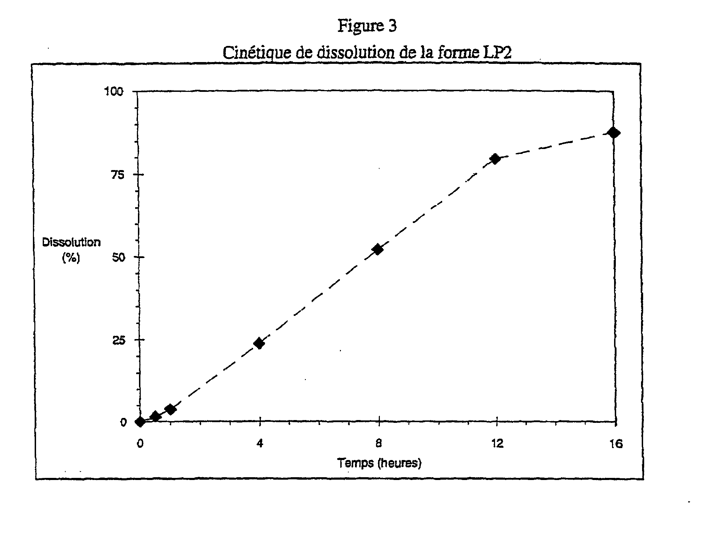

Un comprimé à libération prolongée (LP2) est obtenu suivant le procédé décrit ci-dessus et avec la formule décrite dans le tableau 2. Ce comprimé est ensuite enrobé dans une turbine perforée Manesty de type Accela Cota 10 au moyen d'une suspension aqueuse d'éthylcellulose (Aquacoat® ) renfermant du dibutyl sébaçate utilisé comme plastifiant. Le profil de dissolution in vitro de cette forme (LP2) est présenté dans la figure 3 (annexe).

Avec cette forme LP2, le temps de latence de la cinétique de dissolution in vitro du principe actif est inférieur à une heure. La vitesse de libération est plus élevée dans cet exemple. L'utilisation d'une suspension aqueuse d'éthylcellulose à la place d'une solution organique permet d'obtenir un profil de libération un peu différent.

### Minigranules :

La préparation des minigranules à libération prolongée est réalisée suivant deux procédés :

### Procédé par extrusion et sphéronisation :

### Stade A

Mélange du dichlorhydrate de trimétazidine avec de la cellulose microcristalline puis mouillage au moyen d'eau purifiée. La masse humide est ensuite granulée puis extrudée au moyen d'une extrudeuse. Les extrudats sont ensuite sphéronisés et séchés.

### Stade B

Enrobage des minigranules obtenus au stade A au moyen d'une solution organique ou d'une suspension aqueuse de polymère pouvant également renfermer un plastifiant, dans une turbine d'enrobage ou un appareil à lit d'air fluidisé.

### Stade C

Mise en gélule des minigranules enrobés.

### Procédé par montage:

### Stade A

Enrobage de minigranules neutres composés par exemple de saccharose, ou de saccharose et d'amidon, ou de cellulose microcristalline au moyen d'une solution aqueuse de principe actif pouvant renfermer un liant tel la polyvidone ou la méthylhydroxypropylcellulose. Cet enrobage peut être réalisé en turbine d'enrobage ou dans un appareil à lit fluidisé.

### Stade B

Le stade B est identique au stade B du procédé par extrusion et sphéronisation.

### Stade C

Mise en gélules des minigranules enrobés.

### Exemple 3 :

Une gélule renfermant des minigranules à libération prolongée (LP3) est préparée en utilisant la formule donnée dans le tableau 3, suivant le procédé opératoire par extrusion et sphéronisation.

**Tableau 3**

| Formule de la gélule LP3 | |
|---|---|
| Constituants | Quantité (mg) |
| Dichlorhydrate de trimétazidine | 80 |
| Cellulose microcristalline | 80 |
| Ethylcellulose | 40 |
| Acétyltributylcitrate | 4 |

Les minigranules obtenus au stade A sont enrobés avec une solution alcoolique d'éthylcellulose (polymère contrôlant la libération du principe actif) additionnée d'acétyltributylcitrate utilisé comme plastifiant.

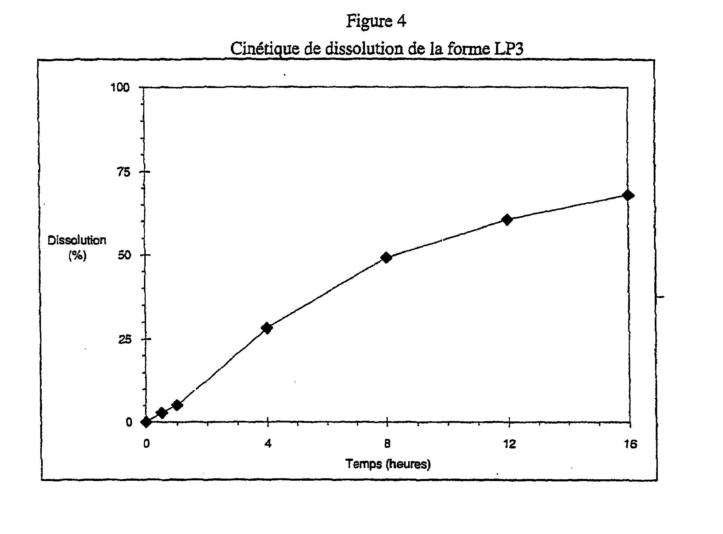

Le profil de dissolution in vitro de cette forme (LP3) est présenté dans la figure 4 (annexe).

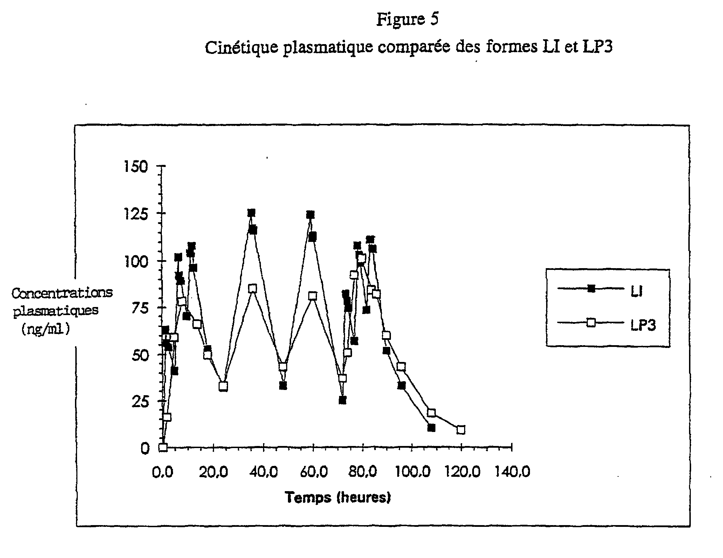

Les concentrations plasmatiques en trimétazidine ont été mesurées chez douze sujets après administration soit d'une gélule LP3 par jour, soit d'un comprimé à libération immédiate (LI) trois fois par jour. La courbe des concentrations plasmatiques moyennes est donnée dans la figure 5 (annexe).

### Exemple 4 :

Une gélule renfermant des minigranules à libération prolongée (LP4) est préparée en utilisant la formule donnée dans le tableau 4, suivant le procédé opératoire par montage.

**Tableau 4**

| Formule de la gélule LP4 | |
|---|---|
| Constituants | Quantités (mg) |
| Dichlorhydrate de trimétazidine | 80 |
| Granule neutre saccharose - amidon | 74 |
| Méthylhydroxypropyl cellulose | 6 |
| Ethylcellulose | 16 |
| Acétyltributyl citrate | 1.6 |

Les minigranules obtenus au stade A sont enrobés avec une solution alcoolique d'éthylcellulose additionnée d'acétyltributyl citrate.

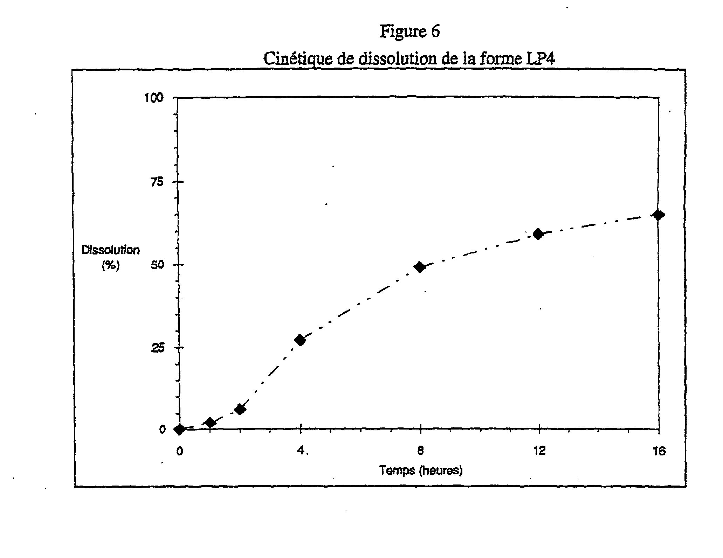

Le profil de dissolution de la forme LP4 est présenté dans la figure 6 (annexe).

### Exemple 5 :

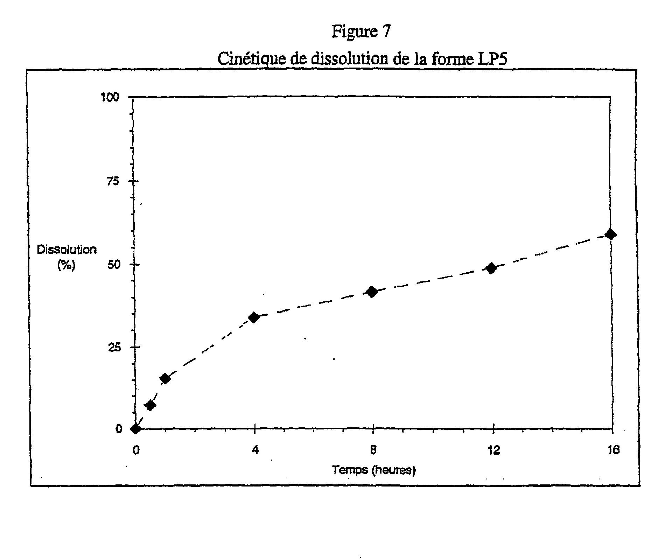

Une gélule LP5 est préparée en utilisant le procédé décrit pour la gélule du type LP3 (exemple 3) dans laquelle l'enrobage au moyen d'une solution alcoolique d'éthylcellulose est remplacé par un enrobage au moyen d'une suspension aqueuse d'éthylcellulose (Aquacoat® ). Son profil de dissolution in vitro est présenté dans la figure 7 (annexe).

Le temps de latence n'existe pas. La cinétique de dissolution in vitro est linéaire après 4 heures.

### Exemple 6 :

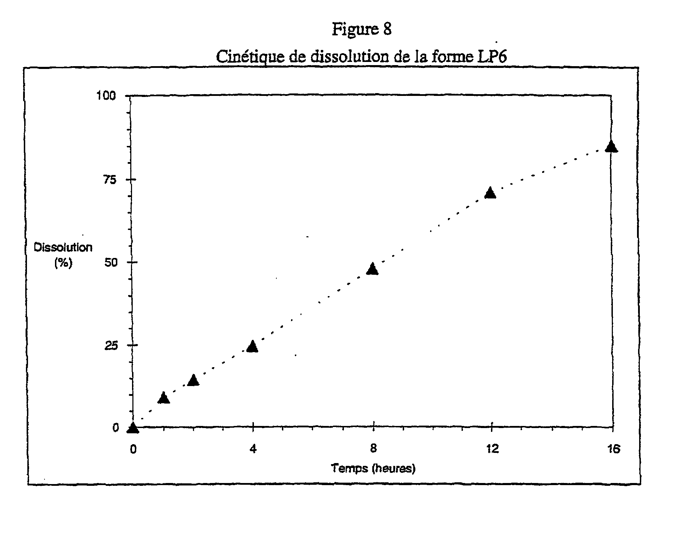

Une gélule du type LP6 est préparée en utilisant le procédé décrit pour la gélule LP4 dans laquelle l'enrobage au moyen d'une solution alcoolique d'éthylcellulose est remplacé par un enrobage au moyen d'une dispersion aqueuse de poly(methy)acrylates (Eudragit® ). Son profil de dissolution in vitro est présenté dans la figure 8 (annexe).

Ainsi, avec l'enrobage au moyen d'une suspension aqueuse d'Eudragit® , il n'existe pas de temps de latence. La cinétique de libération in vitro du principe actif est quasiment linéaire sur 12 heures.

## Revendications

1. Composition pharmaceutique pour la libération prolongée de trimétazidine ou d'un de ses sels pharmaceutiquement acceptable **caractérisée en ce que** la libération prolongée de la trimétazidine est contrôlée par l'utilisation d'un système réservoir dans lequel un mélange de polymère insoluble dans l'eau et d'un plastifiant se présente sous la forme d'un film qui enrobe des comprimés ou des minigranules contenant la trimétazidine ou l'un de ses sels d'addition à un acide pharmaceutiquement acceptable, étant entendu que le plastifiant est différent d'une huile hydrogénée.

2. Composition pharmaceutique selon la revendication 1 dans laquelle la trimétazidine se trouve sous la forme d'un dichlorhydrate.

3. Composition pharmaceutique selon la revendication 1 telle que le système réservoir est un comprimé réservoir.

4. Composition pharmaceutique selon la revendication 1 telle que le système réservoir est une gélule réservoir contenant des minigranules enrobés.

5. Composition pharmaceutique selon la revendication 1 telle que le polymère utilisé est de l'éthylcellulose sous forme de solution organique ou de dispersion aqueuse.

6. Composition pharmaceutique selon la revendication 1 telle que le polymère utilisé est un dérivé acrylique.

7. Composition pharmaceutique selon la revendication 1 telle que le plastifiant est de l'acétyltributylcitrate.

8. Composition pharmaceutique selon la revendication 1 telle que le plastifiant est du dibutylsébaçate.

9. Composition pharmaceutique selon la revendication 1 **caractérisée en ce qu'**elle permet d'obtenir une libération contrôlée et régulière de trimétazidine dès le début de l'administration et sur une durée d'au moins 16 heures.

10. Composition pharmaceutique selon la revendication 1 utile dans le traitement prophylactique de la crise d'angine de poitrine, lors des atteintes choriorétiennes ainsi que lors du traitement des vertiges d'origine vasculaire.

## Claims

1. Pharmaceutical composition for the prolonged release of trimetazidine or of a pharmaceutically acceptable salt thereof, **characterised in that** the prolonged release of trimetazidine is controlled by using a reservoir system in which a mixture of polymer that is insoluble in water and a plasticizer is in the form of a film that coats tablets or minigranules comprising trimetazidine or an addition salt thereof with a pharmaceutically acceptable acid, it being understood that the plasticizer is other than a hydrogenated oil.

2. Pharmaceutical composition according to claim 1, in which the trimetazidine is in the form of a dihydrochloride.

3. Pharmaceutical composition according to claim 1, wherein the reservoir system is a reservoir tablet.

4. Pharmaceutical composition according to claim 1, wherein the reservoir system is a reservoir gelatin capsule comprising coated minigranules.

5. Pharmaceutical composition according to claim 1, wherein the polymer used is ethylcellulose in the form of an organic solution or aqueous dispersion.

6. Pharmaceutical composition according to claim 1, wherein the polymer used is an acrylic compound.

7. Pharmaceutical composition according to claim 1, wherein the plasticizer is acetyl tributyl citrate.

8. Pharmaceutical composition according to claim 1, wherein the plasticizer is dibutyl sebacate.

9. Pharmaceutical composition according to claim 1, **characterised in that** it enables a controlled consistent release of trimetazidine from the moment of administration and for a duration of at least 16 hours.

10. Pharmaceutical composition according to claim 1 for use in the prophylactic treatment of an angina pectoris attack, in chorioretinal attacks and in the treatment of vertigo of vascular origin.

## Patentansprüche

1. Pharmazeutische Zubereitung für die verlängerte Freisetzung von Trimetazidin oder eines seiner pharmazeutisch annehmbaren Salze, **dadurch gekennzeichnet, daß** die verlängerte Freisetzung von Trimetazidin durch die Verwendung eines Speichersystems gesteuert wird, bei dem eine Mischung aus einem in Wasser unlöslichen Polymer und eines Weichmachers in Form eines Films vorliegt, welcher Tabletten oder Minikörnchen, die Trimetazidin oder eines seiner Additionssalze mit einer pharmazeutisch annehmbaren Säure enthalten, umhüllt, wobei es sich versteht, daß der Weichmacher von einem hydrierten Öl verschieden ist.

2. Pharmazeutische Zubereitung nach Anspruch 1, worin Trimetazidin in Form des Dihydrochlorids vorliegt.

3. Pharmazeutische Zubereitung nach Anspruch 1, worin das Speichersystem eine Speichertablette ist.

4. Pharmazeutische Zubereitung nach Anspruch 1, worin das Speichersystem eine Speichergelkapsel ist, welche umhüllte Minikörnchen enthält.

5. Pharmazeutische Zubereitung nach Anspruch 1, worin das verwendete Polymer Ethylcellulose in Form einer organischen Lösung oder einer wäßrigen Dispersion ist.

6. Pharmazeutische Zubereitung nach Anspruch 1, worin das verwendete Polymer ein Acrylderivat ist.

7. Pharmazeutische Zubereitung nach Anspruch 1, worin der Weichmacher Acetyltributylcitrat ist.

8. Pharmazeutische Zubereitung nach Anspruch 1, worin der Weichmacher Dibutylsebacat ist.

9. Pharmazeutische Zubereitung nach Anspruch 1, **dadurch gekennzeichnet, daß** sie die gesteuerte und regelmäßige Freisetzung von Trimetazidin vom Beginn der Verabreichung bis zu einer Dauer von mindestens 16 Stunden ermöglicht.

10. Pharmazeutische Zubereitung nach Anspruch 1 zur prophylaktischen Behandlung von Angina pectoris-Krisen, von chorioretinalen Vorfällen sowie für die Behandlung von gefäßbedingten Schwindelsyndromen.
